# EUROPÄISCHE PATENTANMELDUNG

(11) **EP 4 553 064 A1**
(43) Veröffentlichungstag der Anmeldung: **14.05.2025**
(21) Anmeldenummer: 23209408.6
(22) Anmeldetag: 13.11.2023
(51) Int. Cl.: C07C 67/08, C07C 69/30

(54) **VERFAHREN ZUR HERSTELLUNG VON GLYCERINTRIESTERN**

(71) Anmelder: Evonik Oxeno GmbH & Co. KG, 45772 Marl (DE)
(72) Erfinder: GRASS, Michael, 45721 Haltern am See (DE); KRAFT, Johannes, 63067 Offenbach (DE); VAN EICKELS, Michael, 45657 Recklinghausen (DE); FLEISCHER, Vinzenz, 45770 Marl (DE); BECKER, Stephan, 45770 Marl (DE); BERGUP, Lena, 48734 Reken (DE); WOELK-FÄHRMANN, Michael, 45768 Marl (DE)
(74) Vertreter: Evonik Patent Association

(57) **Zusammenfassung**

Gegenstand der Erfindung ist ein Verfahren zur Herstellung von Glycerinestern der allgemeinen Formel (1) 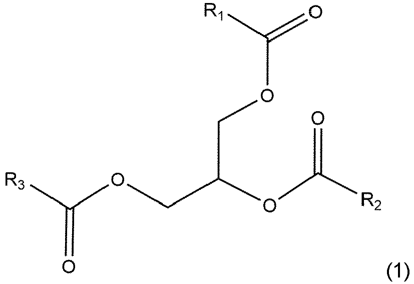 wobei R₁, R₂ und R₃ jeweils unabhängig voneinander aus einer C3- bis C5-Alkylgruppe ausgewählt sind,
durch Veresterung von Glycerin mit einer C4- bis C6-Monocarbonsäure oder einer Mischung von zwei oder mehr C4- bis C6-Monocarbonsäuren.

## Beschreibung

Die vorliegende Erfindung betrifft ein Verfahren zur Herstellung von Glycerinestern der allgemeinen Formel (1) 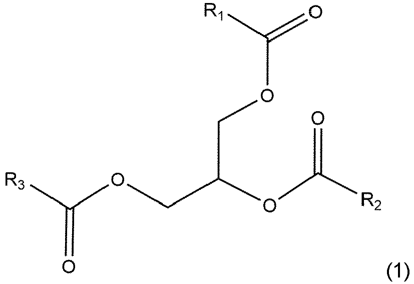
wobei R₁, R₂ und R₃ jeweils unabhängig voneinander aus einer C3- bis C5-Alkylgruppe ausgewählt sind,
durch Veresterung von Glycerin mit einer C4- bis C6-Monocarbonsäure oder einer Mischung von zwei oder mehr C4- bis C6-Monocarbonsäuren.

Glycerinester, insbesondere Tributansäureglycerinester (Glycerintributyrat), sind bekannte chemische Stoffe, die als biobasierte Additive in Kunststoffen diskutiert werden. Biobasiert bedeutet in diesem Zusammenhang, dass sie mit nachwachsenden Rohstoffen hergestellt werden können. Derartige Additive werden in Zukunft an Bedeutung gewinnen, was die großtechnische Herstellung entsprechender Additive notwendig macht.

Die Aufgabe der vorliegenden Erfindung war deshalb die Bereitstellung eines Verfahrens zur Herstellung von Glycerinestern mit C3- bis C5-Alkylgruppen, mit dem die großtechnische Produktion dieser Glycerinester möglich ist.

Die Aufgabe konnte mit dem in Anspruch 1 genannten Verfahren gelöst werden. Bevorzugte Ausführungsformen sind in den Unteransprüchen angegeben.

Das Verfahren zur Herstellung von Glycerinestern der allgemeinen Formel (1) 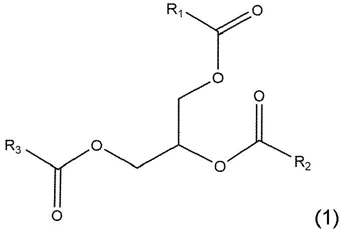
wobei R₁, R₂ und R₃ jeweils unabhängig voneinander aus einer C3- bis C5-Alkylgruppe ausgewählt sind,
umfasst dabei die folgenden Schritte:
   a) Bereitstellen von Glycerin und einer C4- bis C6-Monocarbonsäure oder einer Mischung von zwei oder mehr C4- bis C6-Monocarbonsäuren;
   b) Verestern von Glycerin und der C4- bis C6-Monocarbonsäure oder einer Mischung von zwei oder mehr C4- bis C6-Monocarbonsäuren in einer Reaktionszone, die einen Reaktionsteil und einen Destillationsteil umfasst, in Abwesenheit oder Gegenwart eines Katalysators, wobei das entstehende Reaktionswasser während der Veresterung über den Destillationsteil zumindest teilweise entfernt wird;
   c) Abdestillieren des restlichen Reaktionswassers und Abdestillieren der überschüssigen Monocarbonsäure mit 4 bis 6 Kohlenstoffatomen im Destillationsteil unter Vakuum.

Das erfindungsgemäße Verfahren ist einfach gehalten und ist ohne Probleme großtechnisch einsetzbar.

Die allgemeine Reaktionsgleichung lautet wie folgt: 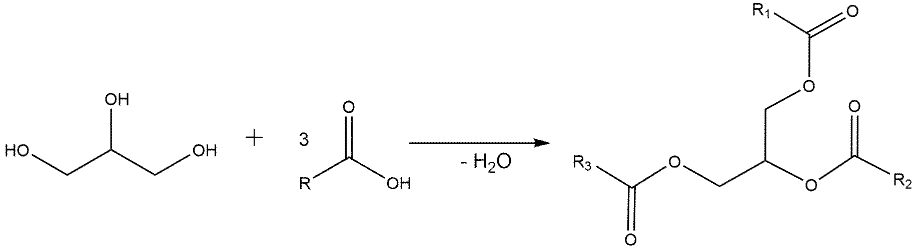 wobei R, R₁, R₂ und R₃ jeweils unabhängig voneinander aus einer C3- bis C5-Alkylgruppe ausgewählt sind.

Das Glycerin wird mit einer C4- bis C6-Monocarbonsäure oder einer Mischung von zwei oder mehr C4- bis C6-Monocarbonsäuren unter Abspaltung von Wasser verestert. Der Rest R entspricht dabei einer C3- bis C5-Alkylgruppe, weil ein C-Atom zur Säuregruppe gehört und sich insgesamt eine C4- bis C6-Monocarbonsäure ergibt. Entsprechend sind die C3- bis C5-Alkylgruppen aus der Monocarbonsäure auch im entstandenen Glycerinester vorhanden.

Schritt a) des erfindungsgemäßen Verfahrens ist die Bereitstellung von Glycerin und einer C4- bis C6-Monocarbonsäure oder einer Mischung von zwei oder mehr C4- bis C6-Monocarbonsäuren. Glycerin ist großtechnisch verfügbar und kann in geeigneten Mengen am Markt bezogen werden. Pro Jahr werden etwa 2 Mio. Tonnen Glycerin auf unterschiedliche Weise produziert darunter auch biobasiertes Glycerin beispielsweise aus der Biodiesel-Herstellung. Erfindungsgemäß ist das bei dem erfindungsgemäßen Verfahren eingesetzte Glycerin ein biobasiertes Glycerin.

Die C4- bis C6-Monocarbonsäuren, die neben dem Glycerin in Schritt a) bereitgestellt werden, sind ebenso großtechnisch verfügbar und können chemisch auf unterschiedliche Weise hergestellt oder isoliert werden. Als C4- bis C6-Monocarbonsäure kann im erfindungsgemäßen Verfahren Butansäure, Isobutansäure, Valeriansäure, Isovaleriansäure (3-Methylbutansäure), 2-Methylbutansäure, Pivalinsäure, Capronsäure (n-Hexansäure), Isocapronsäure (4-Methylpentansäure), 3-Methylpentansäure, 2-Methylpentansäure, 2,2-Dimethylbutansäure, 3,3-Dimethylbutansäure, 2,3-Dimethylbutansäure, 2-Ethylbutansäure oder eine Mischung von zwei oder mehr der genannten C4- bis C6-Monocarbonsäuren eingesetzt werden. Bevorzugte C4- bis C6-Monocarbonsäuren sind Valeriansäure, Isovaleriansäure (3-Methylbutansäure), 2-Methylbutansäure, Isobutansäure und Capronsäure. Sofern eine Mischung von zwei oder mehr C4- bis C6-Monocarbonsäuren eingesetzt wird, ist es bevorzugt, dass eine Mischung von zwei oder mehr C4- bis C6-Monocarbonsäuren mit der identischen Anzahl an Kohlenstoffatomen eingesetzt wird.

Eine bevorzugte Mischung ist eine Mischung aus mindestens einer C4-Monocarbonsäure und mindestes einer C5-Monocarbonsäure. Eine besonders bevorzugte Mischung in diesem Zusammenhang ist eine Mischung aus Valeriansäure, Isovaleriansäure (3-Methylbutansäure), 2-Methylbutansäure und Isobutansäure, die insbesondere 5 bis 50 Gew.-% Valeriansäure, 1 bis 30 Gew.-% 2-Methylbutansäure, 5 bis 35 Gew.-% Isovaleriansäure (3-Methylbutansäure) und 10 bis 50 Gew.-% Isobutansäure enthält, wobei die Angabe Gew.-% jeweils auf die gesamte Mischung bezieht und sich die einzelnen Werte immerzu 100% addieren müssen.

In einer bevorzugten Ausgestaltung der vorliegenden Erfindung wird eine C5-Monocarbonsäure oder eine Mischung von C5-Monocarbonsäuren in Schritt a) bereitgestellt und mit dem Glycerin in Schritt b) verestert. Als C5-Monocarbonsäure kommen insbesondere Valeriansäure, Isovaleriansäure (3-Methylbutansäure) oder 2-Methylbutansäure in Betracht. Als Mischung von C5-Monocarbonsäuren kann demnach eine Mischung aus Valeriansäure und/oder Isovaleriansäure (3-Methylbutansäure) und/oder 2-Methylbutansäure eingesetzt werden.

Eine besonders bevorzugte Mischung von C5-Monocarbonsäuren ist eine Mischung aus Valeriansäure und 2-Methylbutansäure, die 10 bis 90 Gew.-%, vorzugsweise 40 bis 80 Gew.-% Valeriansäure und 10 bis 90 Gew.-%, vorzugsweise 20 bis 60 Gew.-% 2-Methylbutansäure enthält.

Glycerin hat 3 Alkoholgruppen und soll daher mit 3 C4- bis C6-Monocarbonsäuren zum Triester reagieren. Die C4- bis C6-Monocarbonsäure oder die Mischung von zwei oder mehr C4- bis C6-Monocarbonsäuren muss daher zumindest in einer Menge bereitgestellt werden, dass 3 Mol C4-bis C6-Monocarbonsäure(n) pro 1 Mol Glycerin vorhanden sind. Erfindungsgemäß ist es bevorzugt, wenn die C4- bis C6-Monocarbonsäure(n) im leichten stöchiometrischen Überschuss eingesetzt werden, d. h. > 3 Mol C4- bis C6-Monocarbonsäure(n) pro 1 Mol Glycerin, insbesondere 3,1 bis 4,1 Mol C4- bis C6-Monocarbonsäure(n) pro 1 Mol Glycerin vorhanden sind.

Im nachfolgenden Schritt b) werden Glycerin und die C4- bis C6-Monocarbonsäure oder die Mischung von zwei oder mehr C4- bis C6-Monocarbonsäuren in einer Reaktionszone, die einen Reaktionsteil und einen Destillationsteil umfasst, verestert. Da die Reaktionslösung mit der C4- bis C6-Monocarbonsäure oder der Mischung von zwei oder mehr C4- bis C6-Monocarbonsäuren bereits Säure enthält kann die Reaktion auch ohne zusätzlichen Katalysator, d. h. autokatalysiert, durchgeführt werden.

Die Veresterung in Schritt b) kann natürlich auch in Anwesenheit eines Katalysators durchgeführt werden. Grundsätzlich eignen sich saure Katalysatoren dafür, die auch bei anderen Veresterungsreaktionen eingesetzt werden, insbesondere Brönsted- oder Lewis-saure Veresterungskatalysatoren. Bevorzugt wird bei der Veresterung in Schritt b) ein Alkyltitanat, beispielsweise Tetrabutyltitanat; eine Zinkverbindung, beispielsweise ZnCl₂ oder Zn(AcO)₂; oder eine Sulfonsäure, beispielsweise Methansulfonsäure oder Toluolsulfonsäure eingesetzt. Der Katalysator wird in katalytischen Mengen zugegeben, vorzugsweise im Bereich von 0,001 bis 0,01 Mol Katalysator pro Mol Glycerin.

Die Veresterung in Schritt b) des erfindungsgemäßen Verfahrens wird vorzugsweise bei erhöhter Temperatur durchgeführt. Die Reaktionstemperatur bei der Veresterung in Schritt b) liegt vorzugsweise im Bereich zwischen 100 °C und 250 °C, besonders bevorzugt im Bereich zwischen 150 °C und 230 °C. Auch oberhalb und unterhalb dieser Temperaturen kann die Veresterung stattfinden. Allerdings verläuft die Reaktion bei niedrigeren Temperaturen zu langsam und bei höheren Temperaturen besteht eine erhöhte Wahrscheinlichkeit zur Nebenproduktbildung. Hinzu kommt, dass die C4- bis C6-Monocarbonsäuren vergleichsweise niedrige Siedepunkte haben, weshalb die Temperatur bei Normaldruck nicht zu hoch sein sollte, um nicht Energie durch das Verdampfen der Säuren direkt wieder auszutragen.

Der Druck bei der Veresterung in Schritt b) ist weniger kritisch. So kann die Reaktion ohne zusätzlichen Druck, d. h. bei Umgebungsdruck durchgeführt werden. Grundsätzlich können aber auch Überdrücke von bis zu 10 bar angelegt werden. Da die Anforderungen an Druck und Temperatur während der Reaktion nicht besonders hoch sind, können im Reaktionsteil der Reaktionszone von Schritt b) dem Fachmann bekannte Reaktorsysteme eingesetzt werden. Beispiele für geeignete Reaktorsysteme sind Rührkesselreaktoren, Rührkesselkaskaden oder Rohrreaktoren. Die Veresterung in Schritt b) kann in einem oder mehreren parallel oder in Reihe geschalteten Reaktoren durchgeführt werden.

Während der Veresterung in Schritt b) entsteht - wie bei jeder Veresterungsreaktion - Wasser, das sogenannte Reaktionswasser. Das Wasser wird schon während der Veresterungsreaktion in Schritt b) zumindest teilweise abgetrennt, vorzugsweise als Azeotrop mit der C4- bis C6-Monocarbonsäure oder einer Mischung von zwei oder mehr C4- bis C6-Monocarbonsäuren. Dadurch wird das Gleichgewicht der Reaktion in Richtung Ester verschoben. Die Reaktionszone weist daher einen Destillationsteil auf, um das Reaktionswasser abzutrennen, beispielsweise eine, vorzugsweise am Reaktionsteil angeordnete Destillationskolonne. Zur Verbesserung des gas/flüssig-Stoffaustausches kann die Destillationskolonne Füllkörper oder dem Fachmann geläufige Packungen enthalten. Der Reaktionsteil und der Destillationsteil sind vorzugsweise so miteinander verbunden, dass eine gasförmige Phase aus dem Reaktionsteil in den Destillationsteil und eine flüssige Phase aus dem Destillationsteil in den Reaktionsteil gelangen kann. Bevorzugt umfasst der Destillationsteil eine mit dem Reaktionsteil verbundene Destillationskolonne, durch die vorbeschriebene Verbindung von Reaktionsteil und Destillationsteil sichergestellt wird. Grundsätzlich wäre auch denkbar, dass die Veresterung in Reaktivdestillationskolonnen stattfindet, bei der die Veresterung in der Kolonne stattfindet. Dabei befinden sich Reaktionsteil und Destillationsteil in der Kolonne.

In einer bevorzugten Ausführungsform der vorliegenden Erfindung ist am Kopf der Destillationskolonne am Reaktor eine Vorrichtung vorhanden, mit der der Kopfstrom auskondensiert wird. Das kann ein handelsüblicher Kondensator sein. Möglich ist auch die Kondensationswärme über einen Wärmetauscher für die Beheizung anderer Ströme oder der Reaktion zu nutzen. Durch die Vorrichtung, vorzugsweise den Kondensator, wird der Kopfstrom der Destillationskolonne zumindest teilweise auskondensiert. Das Reaktionswasser kann aus dem so erhaltenen Kondensat abgetrennt werden.

In einer besonders bevorzugten Ausführungsform der vorliegenden Erfindung ist nach der Kondensationsvorrichtung ein Phasentrennvorrichtung angeordnet. Bei der Kondensation werden eine organische Phase, die die C4- bis C6-Monocarbonsäure(n) und optional auch geringe Mengen an Reaktionswasser enthält, und eine wässrige Phase entstehen. Die wässrige Phase enthält vor allem das Reaktionswasser, kann aber auch geringe Mengen der C4- bis C6-Monocarbonsäure enthalten. In der Phasentrennvorrichtung werden die beiden Phasen voneinander getrennt. Die wässrige Phase wird vorzugsweise aus dem Verfahren ausgeschleust. Die organische Phase wird vorzugsweise zum Reaktionsteil und/oder zum Destillationsteil der Reaktionszone zurückgeführt. Aus dem Destillationsteil kann dann auch organische Phase zum Reaktionsteil fließen.

In einer besonders bevorzugten Ausführungsform der vorliegenden Erfindung wird bei der Veresterung in Schritt b) ein zusätzliches aprotisch unpolares Lösemittel hinzugefügt. Das aprotisch unpolare Lösemittel dient dabei als Schleppmittel, mit dem die Trennung der wässrigen und der organischen Phase unterstützt wird. Geeignete Lösemittel sind aprotisch unpolare Lösemittel, die in dem Reaktionssystem inert sind und die mit den Säuren und dem Reaktionswasser unter Reaktionsbedingungen ein ternäres Azeotrop bilden, das nach der Kondensation in eine wässrige und organische Phase zerfällt. Das aprotisch unpolare Lösemittel ist vorzugsweise Cyclohexan oder Toluol. Ein weiterer Vorteil des Schleppmittels ist die beschleunigte Abtrennung des Wassers durch Verdampfen aus dem Reaktionsteil.

Die erfindungsgemäße Veresterung zur Herstellung von Glycerinestern nach der oben gezeigten Formel (1) mit C3- bis C5-Alkylgruppen kann sowohl in kontinuierlicher als auch in Batch-Fahrweise durchgeführt werden. Bevorzugt ist eine Batch-Fahrweise, weil dort die Abtrennung des Reaktionswassers und damit die Vollständigkeit der Reaktion einfacher erreicht werden kann.

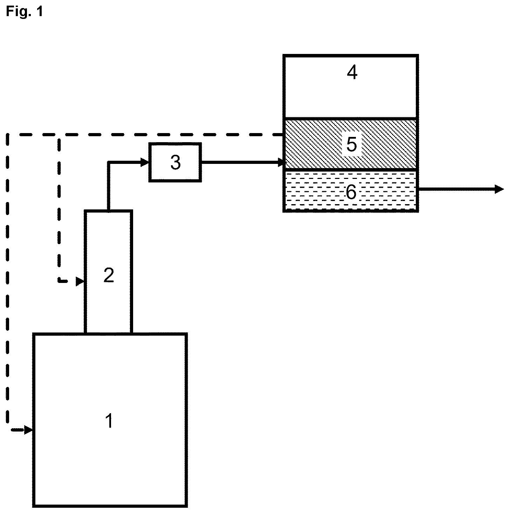

Fig. 1 zeigt eine bevorzugte Ausführungsform der vorliegenden Erfindung. Die Reaktionszone umfasst dabei einen Reaktionsteil (1) und einen Destillationsteil (2), die so miteinander verbunden sind, dass eine gasförmige Phase aus dem Reaktionsteil (1) in den Destillationsteil (2) und eine flüssige Phase aus dem Destillationsteil (2) in den Reaktionsteil (1) gelangen kann. Am Kopf der Destillationseinheit (1) fällt eine gasförmige Phase an, die im Kondensator (3) zumindest teilweise kondensiert wird. Die kondensierte Phase umfasst eine organische Phase (5), die die C4- bis C6-Monocarbonsäure und optional auch geringe Mengen an Reaktionswasser enthält, und eine wässrige Phase (6). In der Phasentrennvorrichtung (4) werden diese beiden Phasen voneinander getrennt, vorzugsweise unter Zuhilfenahme eines unpolar aprotischen Lösemittels wie Cyclohexan. Die wässrige Phase (6) wird aus dem Verfahren ausgeschleust. Die organische Phase (6) wird zur Reaktionszone zurückgeführt. Dabei kann die organische Phase zum Destillationsteil (2) und/oder zum Reaktionsteil zurückgeführt werden.

Die vorliegende Erfindung wird nachfolgend anhand von Beispielen erläutert. Die Beispiele betreffen bevorzugte Ausführungsformen, sind aber nicht als die Erfindung einschränkend zu verstehen.

### Beispiele

Alle Versuche wurden in einem Kolben (500 ml oder 1 I) durchgeführt, an den ein Kondensator mit angeschlossenem Phasentrenner, angeschlossen war. Der Kolben wurde über einen Heizmantel beheizt. Im Kolben befand sich zudem ein Thermoelement zur Kontrolle der Temperatur. Die Versuche wurden zudem unter Stickstoffstrom durchgeführt.

Vor Beginn der Reaktion wurden die Edukte, d. h. das Glycerin, die C4- bis C6-Monocarbonsäure (die jeweils eingesetzte Säure ist in Tabelle 1 zu finden) und optional der Katalysator eingefüllt. Das Gesamtgewicht der Edukte betrug jeweils etwa 450 g. Die Versuche wurden gestartet, in dem die Reaktionslösung aus den Edukten auf eine Temperatur im Bereich von 170 bis 210 °C erhitzt wurde. Über den Kondensator wurden während der Reaktion eine wässrige und eine organische Phase auskondensiert und im Phasentrenner voneinander getrennt. Von Zeit zu Zeit wurde Wasser aus dem Phasentrenner abgelassen. Die organische Phase wurde zurück in den Kolben geleitet. Alle Experimente wurden mit Cyclohexan durchgeführt, um eine bessere Trennung von wässriger und organischer Phase zu erreichen.

Die genauen Angaben der einzelnen Versuche sind in der nachfolgenden Tabelle 1 zu finden.

**Tabelle 1: Übersicht über die Versuchsergebnisse**

| **Bsp.** | **Säure(n)** | **Mol Säure pro Mol Glycerin** | **Katalysator** | **Temperatur / °C** | **Umsatz Triester / %** |
|---|---|---|---|---|---|
| 1 | Valeriansäure | 3,75 | Tetra-n-butyltitanat (Mol Kat/ Mol Glycerin = 0,0015) | 185 | 100 |
| 2 | 2-Methylbutansäure | 3,75 | Tetra-n-butyltitanat (Mol Kat/ Mol Glycerin = 0,0015) | 185 | 96 |
| 3 | 3-Methylbutansäure | 3,75 | Tetra-n-butyltitanat (Mol Kat/ Mol Glycerin = 0,0015) | 185 | 89 |
| 4 | Isobutansäure | 3,75 | Tetra-n-butyltitanat (Mol Kat/ Mol Glycerin = 0,0015) | 185 | 100 |
| 5 | Mischung aus Valeriansäure und 2-Methylbutansäure (Mischungsverhältnis in Mol% in der genannten Reihenfolge 30% : 70%) | 3,75 | - | 185 | 76 |
| 6 | Mischung aus 3-Methylbutansäure und Isobutansäure (Mischungsverhältnis in Mol% in der genannten Reihenfolge 35% : 65%) | 3,75 | - | 185 | 88 |
| 7 | Mischung aus Valeriansäure, 2-Methylbutansäure, 3-Methylbutansäure und Isobutansäure (Mischungsverhältnis in Mol% in der genannten Reihenfolge 33,2% : 14,3% : 19,1% : 33,4%) | 3,75 | Tetra-n-butyltitanat (Mol Kat/ Mol Glycerin = 0,0015) | 185 | 82 |
| 8 | Mischung aus Valeriansäure und 2-Methylbutansäure (Mischungsverhältnis in Mol%in der genannten Reihenfolge 30% : 70%) | 3,75 | Tetra-n-butyltitanat (Mol Kat/ Mol Glycerin = 0,0015) | 185 | 100 |
| 9 | Mischung aus 3-Methylbutansäure und Isobutansäure (Mischungsverhältnis in Mol%in der genannten Reihenfolge 35% : 65%) | 3,75 | Tetra-n-butyltitanat (Mol Kat/ Mol Glycerin = 0,0015) | 185 | 100 |

## Patentansprüche

1. Verfahren zur Herstellung von Glycerinestern der allgemeinen Formel (1) 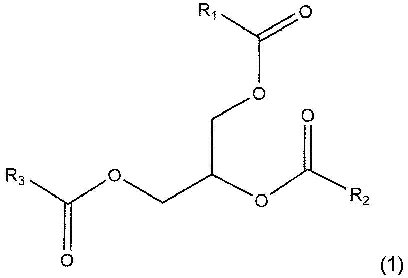
wobei R₁, R₂ und R₃ jeweils unabhängig voneinander aus einer C3- bis C5-Alkylgruppe ausgewählt sind,
wobei das Verfahren die folgenden Schritte umfasst:
a) Bereitstellen von Glycerin und einer C4- bis C6-Monocarbonsäure oder einer Mischung von zwei oder mehr C4- bis C6-Monocarbonsäuren mit der identischen Anzahl an Kohlenstoffatomen;
b) Verestern von Glycerin und der C4- bis C6-Monocarbonsäure oder einer Mischung von zwei oder mehr C4- bis C6-Monocarbonsäuren in einer Reaktionszone, die einen Reaktionsteil und einen Destillationsteil umfasst, in Abwesenheit oder Gegenwart eines Katalysators, wobei das entstehende Reaktionswasser während der Veresterung zumindest teilweise entfernt wird;
c) Abdestillieren des restlichen Reaktionswassers und Abdestillieren der überschüssigen Monocarbonsäure mit 4 bis 6 Kohlenstoffatomen im Destillationsteil unter Vakuum.

2. Verfahren nach Anspruch 1, wobei die C4- bis C6-Monocarbonsäure(n) in Schritt a) im Überschuss eingesetzt werden.

3. Verfahren nach Anspruch 1 oder 2, wobei als C4- bis C6-Monocarbonsäure Butansäure, Isobutansäure, Valeriansäure, Isovaleriansäure, 2-Methylbutansäure, Pivalinsäure, Capronsäure, Isocapronsäure, 3-Methylpentansäure, 2-Methylpentansäure, 2,2-Dimethylbutansäure, 3,3-Dimethylbutansäure, 2,3-Dimethylbutansäure, 2-Ethylbutansäure oder Mischungen von zwei oder mehr davon eingesetzt werden.

4. Verfahren nach Anspruch 3, wobei als C4- bis C6-Monocarbonsäure Valeriansäure, Isovaleriansäure (3-Methylbutansäure), 2-Methylbutansäure, Isobutansäure oder Capronsäure eingesetzt werden.

5. Verfahren nach einem der vorhergehenden Ansprüche, wobei die Veresterung in Schritt b) in Anwesenheit eines Katalysators durchgeführt wird.

6. Verfahren nach einem der vorhergehenden Ansprüche, wobei die Reaktionstemperatur im Bereich zwischen 100 °C und 250 °C, vorzugsweise zwischen 150 °C und 230 °C liegt.

7. Verfahren nach einem der vorhergehenden Ansprüche, wobei die Veresterung in Schritt b) bei Umgebungsdruck durchgeführt wird.

8. Verfahren nach einem der vorhergehenden Ansprüche, wobei ein aprotisch unpolares Lösemittel zur Veresterung in Schritt b) gegeben wird, um die Trennung der wässrigen Phase und der organischen Phase zu befördern.

9. Verfahren nach Anspruch 8, wobei das aprotisch unpolare Lösemittel Cyclohexan ist.

10. Verfahren nach einem der vorhergehenden Ansprüche, wobei die Reaktionsteil und Destillationsteil so miteinander verbunden sind, dass eine gasförmige Phase aus dem Reaktionsteil in den Destillationsteil und eine flüssige Phase aus dem Destillationsteil in den Reaktionsteil gelangen kann.

11. Verfahren nach einem der vorhergehenden Ansprüche, wobei der Destillationsteil eine Destillationskolonne umfasst und wobei der dort anfallende Kopfstrom kondensiert wird.

12. Verfahren nach Anspruch 11, wobei das Kondensat in einer Phasentrennvorrichtung in eine wässrige Phase und eine organische Phase getrennt wird.

13. Verfahren nach Anspruch 12, wobei die wässrige Phase aus dem Verfahren ausgeschleust wird.

14. Verfahren nach Anspruch 12 oder 13, wobei die organische Phase zur Reaktionszone zurückgeführt wird.

15. Verfahren nach einem der vorhergehenden Ansprüche, wobei das Verfahren in Batch-Fahrweise betrieben wird.
